# EUROPEAN PATENT APPLICATION

(11) **EP 1 445 313 A1**
(43) Date of publication of application: **11.08.2004**
(21) Application number: 02801592.3
(22) Date of filing: 17.10.2002
(51) Int. Cl.: C12N 15/11, C12Q 1/68

(54) **METHOD OF DETECTING AND QUANTIFYING HEMOLYSIN-PRODUCING BACTERIA BY OVERWHELMINGLY DETECTING AND QUANTIFYING THERMOSTABLE HEMOLYSIN-RELATED GENES (tdh-RELATED HEMOLYSIN GENES) OF FOOD POISONING BACTERIA**

(30) Priority: 18.10.2001 JP 2001320403
(71) Applicant: NICHIREI CORPORATION, Tokyo 104-8402 (JP)
(72) Inventor: KOIZUMI, Takeshi, c/o Nichirei Corp., R&D Division, Chiba-shi, Chiba 261-8545 (JP); YAMAMOTO, Satoshi, c/o Nichirei Corp.,R&D Division, Chiba-shi, Chiba261-8545 (JP); ITOH, Takeshi, c/o Tokyo Kenbikyo-in Foundation, Nihonbashi, Chuo-ku, Tokyo 103-0015 (JP); NAKAGAWA, Hiroshi, c/o Tokyo Kenbikyo-in Found., Nihonbashi, Chuo-ku, Tokyo 103-0015 (JP)
(74) Representative: Woods, Geoffrey Corlett
(86) International application number: PCT/JP2002/010795
(87) International publication number: WO 2003/033702

(57) **Abstract**

The present invention simply, rapidly and accurately detects, quantitatively determines and types genes of protein groups [which refer to TDH (Thermostable direct hemolysin), TRH (TDH-related hemolysin) and other analogous hemolysin proteins, hereinafter referred to as TDH-related toxins] of thermostable hemolysin groups produced by pathogenic bacteria of the genus *Vibrio*.

Present Invention provides a method which enables detection and quantitative determination of actual TDH-related toxin-producing bacteria, which are important in food hygiene control.

Using a primer pair which contains gene sequences encoding common amino acid sequences of TDH and TRH, TDH-related toxin genes are comprehensively detected and determined, thereby TDH-related toxin-producing bacteria themselves are detected and quantitative determined.

## Description

### Technical Field

The present invention relates to a method for detecting, quantitatively determining, and typing simply, rapidly and accurately the genes of thermostable hemolysin protein groups [which refer to TDH (Thermostable direct hemolysin), TRH (TDH-related hemolysin) and other analogous hemolysin proteins, hereinafter referred to as TDH-related toxins] produced by pathogenic bacteria of the genus *Vibrio*, such as *Vibrio parahaemolyticus*, part of *Vibrio cholerae, Vibrio mimicus* and *Vibrio hollisae*. Namely, the present invention relates to the fields of fishery, food industry, public health, clinical laboratory testing and the like.

### Background Art

Pathogenic bacteria of the genus *Vibrio* including *Vibrio parahaemolyticus*, part of *Vibrio cholerae*, *Vibrio mimicus, Vibrio hollisae* are of an important bacterial species group for food hygiene control. They cause serious symptoms after oral infection and can cause death in patients. In Japan, the incidence of *Vibrio parahaemolyticus* food poisoning is particularly high, and is always a highly ranked among the causes of food poisoning. In this regard, it is necessary to test the main sources of infection, fishes and seafoods to be consumed uncooked, for *Vibrio parahaemolyticus*. Only some genus *Vibrio* bacteria those with the genes of thermostable hemolysin protein (Thermostable direct hemolysin: TDH) and those with the genes of the analogous toxin protein (TDH-related hemolysin: TRH), are causative of food poisoning. At the food hygiene site, food hygiene control is performed by setting an upper limit of tolerance for the total count of *Vibrio parahaemolyticus* existing in food. This is based on the premise that toxin-producing bacteria form a certain proportion or more of *Vibrio parahaemolyticus* isolated from food (perishable fishes and seafoods). Specifically, the number of bacteria suspected to be *Vibrio parahaemolyticus* is counted according to "Food hygiene inspection guidelines." If the number of bacteria is equal to or less than the reference value (100cfu/g or less), the food may be offered (the 22nd notice issued from Standards Division, Department of Food Safety, Pharmaceutical and Food Safety Bureau, Ministry of Health, Labour and Welfare, Japan).

As described above, pathogenic bacteria of *Vibrio parahaemolyticus* are only those capable of producing TDH or TRH. In addition, TDH is also present in bacteria of the genus *Vibrio* other than *Vibrio parahaemolyticus*. Hence, from a broad view point, that is, food poisoning prevention, desirable control should use as an index not the number of *Vibrio parahaemolyticus*, but the presence or absence of, or the number of bacteria capable of producing TDH or TRH.

It has been reported that bacteria of the genus *Vibrio* other than *Vibrio parahaemolyticus*, that is, some strains of *Vibrio chorelae* non-01, *Vibrio hollisae*, and *Vibrio mimicus*, have TDH-producing gene *tdh* (Infect. Immun., 52, 319-322, 1986, J. Bacteriol., 171, 6859-6861, 1989, FEMS Microbiol. L ett., 84, 249-254, 1990). It has been shown that TDH-related toxin protein has evolved independently from the phylum of actual pathogenic bacteria of the genus *Vibrio*, and the pathogenic bacteria of the genus *Vibrio* have acquired each toxin in an independent process by horizontal transmission (J. Bacteriol., 173, 5036-5046, 1991). Therefore, there seems to be a possibility that strains other than known toxin-producing strains have acquired and retained the gene of TDH-related toxin. From this point of view, it can be said that detection and quantitative determination of actual bacteria capable of producing TDH-related toxin protein, done at the site of food hygiene control, is ideal. However, there has been no measures with good versatility and reliability for detecting and quantitatively determining TDH-related toxin, so that detection at the site of food hygiene control has been impossible to execute.

### Disclosure of Invention

### Problems to be Solved by the Invention

As described above, detection and quantitative determination of actual bacteria capable of producing TDH-related toxin protein, done at the site of food hygiene control, is ideal. Total detection of a gene encoding TDH-related toxin protein and quantitative determination of the number of copies are appropriate for this purpose. Detection and quantitative determination of TDH-related toxin protein make possible the detection and quantitative determination of bacteria which may possibly have the same toxins and the same pathogenicity.

However, the technical problems associated with testing by this method are large. Specifically, there has been no method for detecting simultaneously and totally all the types of known genes of TDH-related toxin protein groups. Accordingly, when gene amplification, such as a PCR method using primers, and detection are performed, primer types must be designed individually, and a plurality of primers must be used simultaneously upon testing. As a result, this method is impractical. In addition, as described later, the use of combinations of currently existing primers is highly likely to fail to detect all TDH-related toxin genes.

TDH is hemolysin specified as a causative substance of Kanagawa phenomenon, a phenomenon which causes hemolysis of human red blood cells, and is associated with the pathogenic factor of *Vibrio parahaemolyticus* food poisoning. Based on the result of an animal experiment in which purified TDH showed similar properties with those observed in a case when viable cells were administered, TDH has become recognized as a pathogenic factor. On the other hand, TRH has been discovered in strains derived from patients with food poisoning showing no Kanagawa phenomenon. TRH shows about 68% homology at amino acid level with TDH, and unlike TDH, TRH is thermolabile hemolysin (Infect. Immun., 56, 961-965, 1988).

*trh* gene encoding TRH is known to comprise two types (*trh1* and *trh2*) which differ from each other by about 16% in nucleotide sequence (Appl. Environ. Microbiol., 58, 2449-2457, 1992). In addition, from alignment comparison of respective amino acid sequences of TDH type toxin group and TRH type toxin group, it is inferred that both toxin groups have passed through a long-term evolution process and each has acquired genetic diversity. Therefore, toxin having an unknown intermediate type sequence is likely to be present. Actually, it has been reported that the *tdh* gene of some strains of *Vibrio hollisae* is phylogenetically different from the *tdh* gene of other bacteria of the genus *Vibrio*, such as *Vibrio parahaemolyticus* (Microbiol. Immunol., 40, 59-65, 1996). Furthermore, it is shown in this report that 2 out of 3 existing PCR primer pairs for detection of *tdh* genes failed to detect completely every type of *tdh* gene of tested *Vibrio hollisae*. Moreover, it has been reported regarding *trh* genes that nucleotide sequences of *trh1* and *trh2* genes vary among strains (Appl. Environ. Microbiol., 58, 2449-2457, 1992). However, variation in the nucleotide sequences of *trh1* and *trh2* genes has not been considered in the design of existing PCR primers for detecting *trh* genes, so that it is hard to say that such PCR primers for detecting *trh* are good enough. Actually, in the process of the present invention, a new toxin gene that is analogous to TRH and that is hard to amplify using standard primers was found. These results strongly suggest the possible presence of TDH-related toxin genes other than the existing genes.

Now, PCR primers for detecting TDH-producing gene *tdh* and TRH-producing gene *trh* separately (primers for detecting only *tdh* gene, primers for detecting only *trh1* gene, and primers for detecting *trh1* and *trh2* genes simultaneously) have already been developed for testing toxin genes (Japanese Patent Laid-Open No. 4-293486, Mol. Cell. Probes, 6: 477-487, 1992), and they are on the market as a reagent for studying *Vibrio parahaemolyticus*. However, for the reason described above, none of these commercially available reagents provide a method for totally detecting and quantitatively determining TDH-related toxin or the toxin gene which may exist in food. That is, when such reagents are used at the site of food hygiene control, simultaneous use of a plurality of detection systems is required, and even so, pseudo-negative results remain a possibility.

### Means for Solving the Problems

As a result of studies to solve the above problems, we have found that highly conserved regions are present on TDH and TRH proteins. Specifically, we found, by multi-alignment analysis on known amino acid sequences of TDH and TRH, regions in both TDH and TRH showing high homology. We hereby completed the present invention.

The present invention is characterized in that hemolysin genes *tdh* and *trh* can be simultaneously detected by designing mixed primers (degenerate primers) using a nucleotide sequence which corresponds to the amino acid sequence showing high homology to such two types of hemolysin having different sequences; enabling amplification by the PCR method of fragments of both genes *tdh* and *trh* encoding the 2 types of hemolysin; and obtaining from both the TDH-producing strain and the TRH-producing strain the amplified fragments of a sequence flanked by the primers. Furthermore, the present invention is also characterized in that the detected toxin types can be identified, and fragments of an unknown toxin gene which are functionally analogous to TDH and TRH can be detected.

### Brief Description of Drawings

[Figure 1]

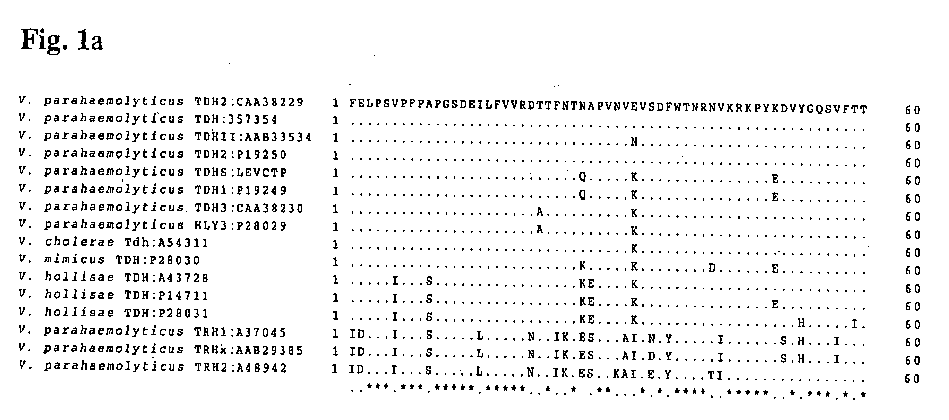

   Fig. 1a-1c. Results of multiple alignment analysis based on existing TDH and TRH amino acid sequences
   The signal peptide sequences (24 amino acids in the first half) in the amino acid sequences of 13 types of TDH (8 types of *V*. *parahaemolyticus*, one type of *V*. *mimicus*, one type of *V*.*cholerae* and 3 types of *V*. *hollisae*) and 3 types of TRH (*V*. *parahaemolyticus* only) that are present on the Entrez Protein database were deleted, and then multiple alignment analysis was performed. Numbers following ':' subsequent to bacterial species name and toxin type denote Accession Nos. of the database.
[Figure 2]

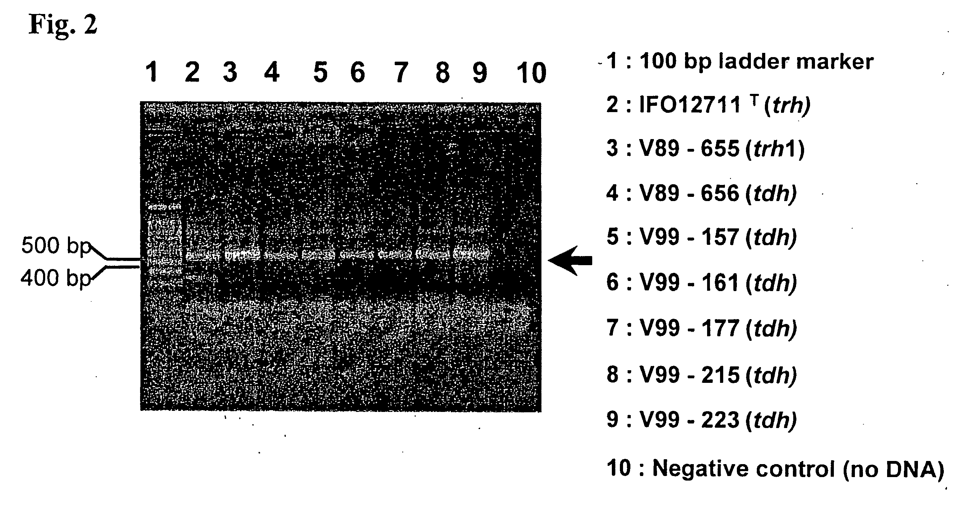

   Fig. 2 Analysis of PCR amplification products by agarose gel electrophoresis lane 1, 100 bp ladder marker; lane 2, IFO 12711 T (*trh*); lane 3, V89 - 655 (*trh1*); lane 4, V89 - 656 (*tdh*); lane 5, V99 - 157 (*tdh*); lane 6, V99 - 161 (*tdh*); lane 7, V99 - 177 (*tdh*); lane 8, V99 - 215 (*tdh*); lane 9, V99 - 223 (*tdh*); lane 10, Negative control (no DNA)
[Figure 3]

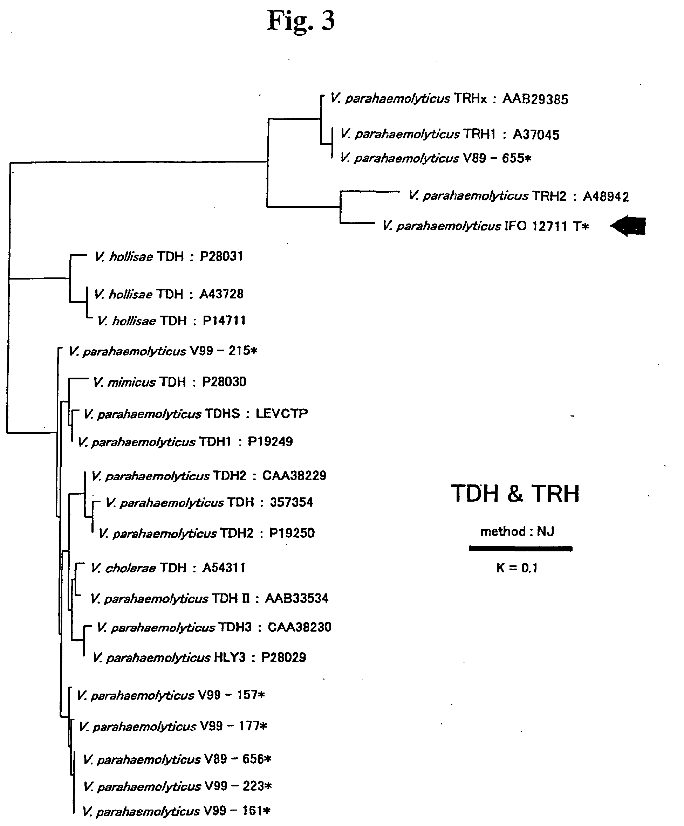

   Fig. 3 Phylogenetic trees of DNA fragment amplified by PCR
   Phylogenetic trees were constructed by the neighbor - joining method (NJ) after translation of the analyzed nucleotide sequences into amino acid sequences. Data analyzed in the present invention are denoted with a symbol *. For data other than those denoted with * , sequences present in the Entrez Protein database were used (in the figure, numbers following ':' denote Accession Nos. on the database).
   TRH contained in the type strain of *Vibrio parahameolyticus* (IFO 12711 T) is denoted with ←.
[Figure 4]

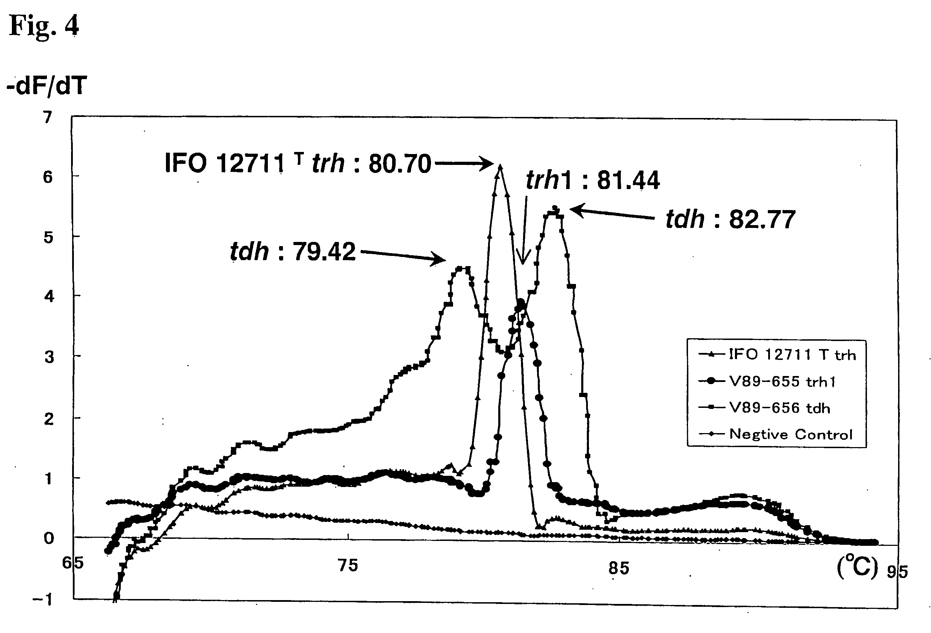

   Fig. 4 Analysis of Tm value of amplified DNA product by melting curve analysis
   PCR products amplified using the light cycler were heated at a rate of 0.1°C per second to a temperature range of 65°C to 95°C, and then the melting curves of the amplified products were analyzed. Light cycler software (version 3) attached to the light cycler was used for analysis.

### Best Mode for Carrying Out the Invention

According to the present invention, bacteria of the genus *Vibrio* which may possibly produce hemolysin are detected, and the toxin type is identified using a mixed nucleic acid sequence encoding a section of a sequence common to both amino acid sequences of TDH and TRH that are encoded respectively by the *tdh* gene and the *trh* gene of some genus *Vibrio* bacteria.

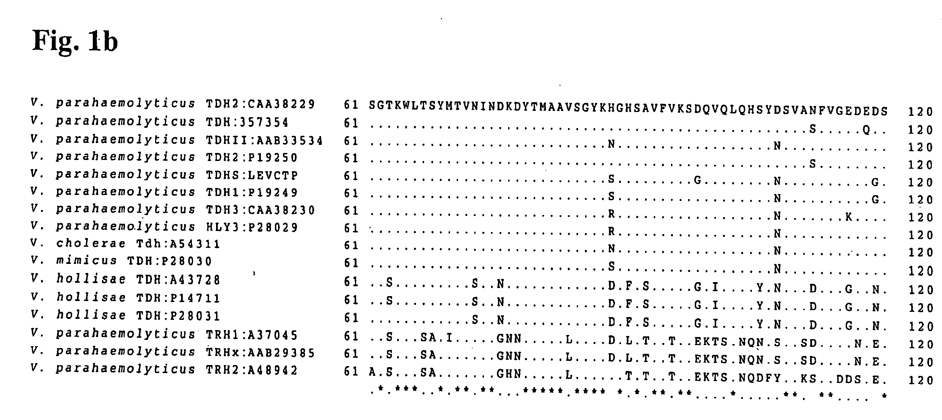

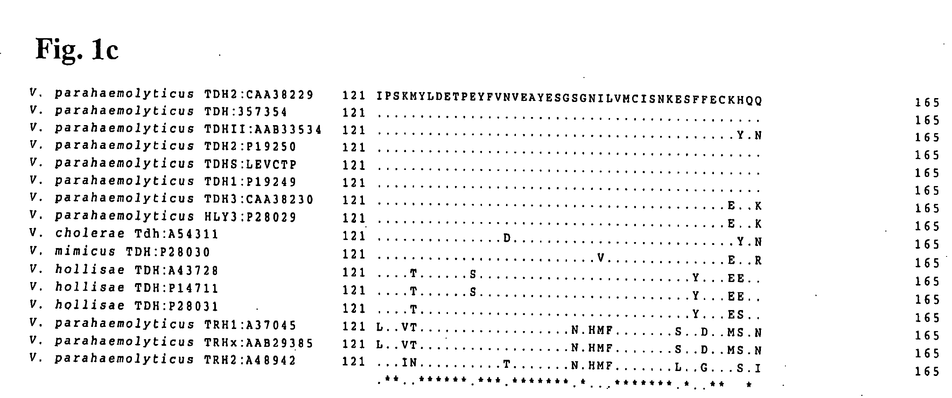

As a result of multiple alignment analysis using the Clustal W computer program on amino acid sequences of 13 types of known thermostable hemolysin TDH and 3 types of TDH-related toxin TRH (Fig. 1), we have found the presence of highly conserved amino acid sequences in the sequences of both toxins. Next, whether or not both *tdh* and *trh* genes can be amplified by the PCR method was studied using the same mixed primers designed by reverse-translating these amino acid sequences into nucleic acid sequences.

Examples of a highly conserved amino acid sequence include (1) LPS (V or I) PFP (A or S) PGSDE (L or I) LFVVR, (2) KRKPY, (3) Y (M or I) TV (N or S) IN, (4) YTMAA (V or L) SGYK, (5) YLDETP (E or S) YFV, (6) VEAYESG and (7) VMCISNK. These sequences respectively correspond to amino acid positions of SEQ ID NO: 1 in Sequence Listing. Specifically, sequence (1) corresponds to amino acid positions 3-21; (2) to 45-49; (3) to 69-75; (4) to 79-88; (5) to 126-135; (6) to 137-143; and (7) to 149-155. (α or β) means either amino acid α or β at the position of the brackets. For example, (5) YLDETP (E or S) YFV stands for either YLDETPEYFV or YLDETPSYFV.

Examples of a particularly preferred highly conserved amino acid sequence include amino acid sequences (1) DE (L or I) LFVV from among LPS (V or I) PFPE (A or S) PGSD (L or I) LFVVLR; (6) VEAYESG; (3) YMTVNIN from among Y (M or I) TV (N or S) IN; and (5) DETPEYFV from among YLDETP (E or S) YFV.

Mixed primers encoding the whole or a part of these amino acid sequences are prepared. Examples of such mixed primers include a sense primer 5'-gaygarhtnytnttygtngt-3' encoding DE (L or I) LFVV and an antisense primer 5'-ccnswytcrtangcytcnac-3' encoding VEAYESG (in the present specification, n denotes a, t, c or g). *tdh* or *trh* gene can be detected easily in a single test by amplifying a sample using these primers.

In addition, genes of the unknown hemolysin which are analogous to above *tdh* and *trh* can be detected by using these primers. In this case, a known sequence is previously added to the 5'terminus of each primer so as to be able to perform direct sequence reaction using amplified fragments. The use of the primers with known sequences added as sequence primers enables direct determination of the nucleotide sequence without cloning the amplified DNA fragment.

The analytical results of the amplified DNA fragments in the present invention can also be evaluated by melting curve analysis or the like in real time PCR analysis without determining nucleotide sequences, as shown in Examples.

The present invention encompasses a kit for detecting, quantitatively determining or identifying a hemolysin gene which uses a combination of mixed primers designed on the basis of the above amino acid sequences, and other reagents.

### Examples

### [Example 1]

Using highly conserved regions shown in Fig. 1, a sense primer 5'-gaygarhtnytnttygtngt-3' encoding (1) DE(L or I)LFVV from among LPS (V or I) PFP (A or S) PGSDE (L or I) LFVVLR, and an antisense primer 5'-ccnswytcrtangcytcnac-3' encoding (6) VEAYESG were designed. These primers respectively correspond to amino acid positions in TDH of *Vibrio parahaemolyticus*, 38-44, and 161-167. There are 3072 combinations of the former mixed primers; and 2048 combinations of the latter mixed primer. An amplified DNA fragment of 386bp can be obtained by PCR using the combination of the primers. However, the obtained DNA fragment can be an unknown hemolysin gene. Therefore, known sequences (sense side: 5'-c aggaaacagctatgacc-3' and antisense side: 5'-tgtaaaacgacggccagt-3') were previously added to the 5' terminus of each primer so as to be able to perform direct sequence reaction using amplified fragments. The use of the primers with known sequences added as sequence primers enables direct determination of the nucleotide sequence without cloning the amplified DNA fragment. In addition, the amplified DNA product is 422bp after addition of this sequence.

Toxin gene fragments were amplified by PCR using the above primers and using as templates the chromosome DNA extracted according to a standard method from 8 strains of *Vibrio parahaemolyticus* listed in Table 1 which are known to produce TDH or TRH.
Amplification reaction was performed using thermostable DNA polymerase (AmpliTaq Gold: Applied Biosystems) and a GENE MATE thermal cycler (ISC BioExpress). A reaction solution was prepared to have a final volume of 50µl from a solution containing DNA 0.1µg, 50mM KCl, 10 mM Tris-HCl (pH 8.3), 2.0 mM MgCl₂, 0.01% gelatin, dNTP (0.2 mM each), 2.5 U of AmpliTaq Gold and primers (1µ M each). A reaction condition consisting of activation with AmpliTaq Gold (95°C for 10 min); followed by 5 reaction cycles of 95°C for 1 min, 45°C for 40 sec and 72°C for 1 min and 30 sec; 35 reaction cycles of 94°C for 1 min, 46°C for 40 sec and 72°C for 1 min and 30 sec; and then elongation reaction at 72°C for 10 min was performed. The resulting PCR products were subjected to 1% agarose gel (Sea Plaque GTG agarose: BioWhittaker Molecular Applications) electrophoresis (0.5xTAE, 100V for 30 min), and then stained with ethidium bromide for 10 min. The presence of amplified products was confirmed under ultraviolet irradiation.

As a result, amplification of DNA fragments of 422bp was confirmed for DNAs of all the strains producing TDH or TRH as shown in Fig. 2.

After these amplified fragments were excised from gel and purified according to a standard method, the nucleotide sequences were determined by a dideoxy method using the above-mentioned sequence primers.

The nucleotide sequence was determined using ABI PRISM BigDye Terminator Cycle Sequenceing Ready Reaction Kit (Applied Biosystems) and ABI PRISM 310 GENETIC ANALYZER (Applied Biosystems).

The resulting nucleotide sequences (SEQ ID NO: 41-46) were translated into amino acid sequences, and confirmed to be genes encoding TDH or TRH.

It was also shown that a toxin gene of *Vibrio parahaemolyticus* type strain (IFO12711 T), which had been so far reported to have *trh2* gene (Summary of *Vibrio parahaemolyticus* Symposium No. 33, 1999: Clinical Microbiology, 27, p239, 2000), is a new gene (SEQ ID NO:40) that differs from *trh2* in sequence (82% homology at amino acid level, and 91% homology at nucleic acid level), and differs from those known to date (Fig. 3).

As a result, simultaneous detection of *tdh* and *trh* genes and detection of unknown TDH-related toxin genes are shown to be possible by the use of mixed primers based on amino acid sequences of highly homologous regions of TDH and TRH proteins.

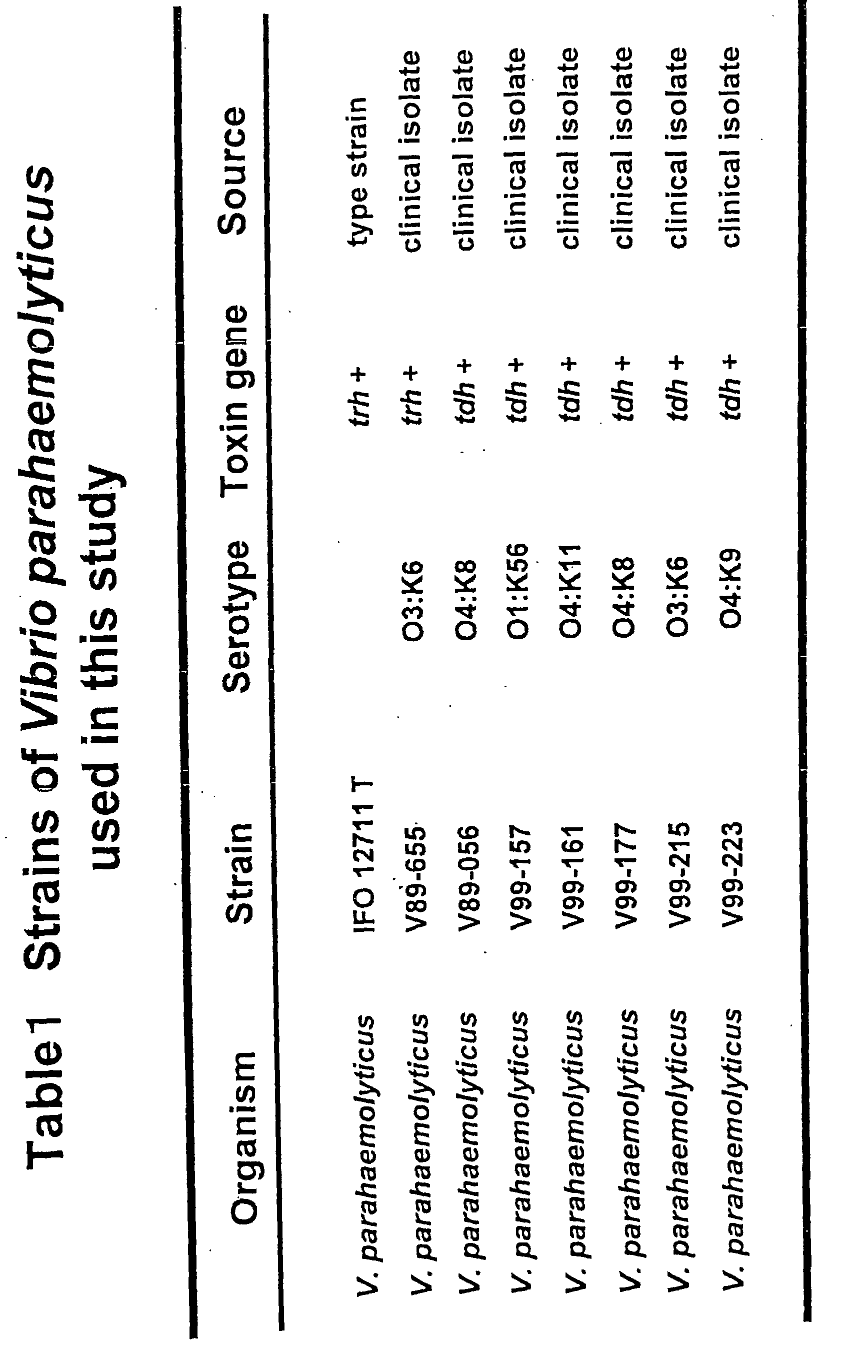

### [Example 2]

A sense primer 5'-tayatgacngtnaayathaayg-3' encoding YMTVNIN among Y(M or I)TV(N or S)IN and an antisense primer 5'-acraartaytcnggngtytcrtc-3' encoding DETPEYFV among YLDETP(E or S)YFV were designed from highly homologous regions shown in Fig. 1. The primers correspond to amino acid positions 100-106 and 152-159, respectively in *Vibrio parahaemolyticus* TDH. Both primers are mixed primers for which there are 512 combinations. An amplified DNA fragment of 180bp can be obtained by PCR using combinations of these primers.

Detection and identification of a hemolysin gene were attempted by the real time PCR using as templates chromosome DNAs derived from a TDH-producing strain, a type 1 TRH-producing strain and *Vibrio parahaemolyticus* type strain (IFO12711 T) that was shown by the present invention to produce a novel TRH, and using LightCycler System (Roche Diagnostics Co., Ltd.). LightCycler - DNA Master SYBR Green I kit (Roche Diagnostics Co., Ltd.) was used as a reaction solution. The reaction solution was prepared at a final volume of 20 µl by addition of 0.1 µg of DNA. To perform automatic hot start, Light Cycler DNA master mix SYBR green I was inactivated by previously reacting with anti-Taq polymerase antibody (Taq Start Antibody : Clontech).

A reaction condition consisting of activation of Taq polymerase (95°C for 90 sec); 5 reaction cycles of 95°C for 0 sec, 44°C for 10 sec, and 72°C for 30 sec; and then 35 reaction cycles of 94°C for 0 sec, 45°C for 10 sec and 72°C for 30 sec was performed. After amplification reaction, the temperature was raised at a rate of 0.1°C per sec from 65°C to 95°C, and then melting curves of amplified products were analyzed.

Amplified products were confirmed from all the DNAs by analysis. Further, as a result of melting curve analysis, *tdh* gene fragment, *trh1* gene fragment and a new *trh* gene fragment all showed different Tm values (specific melting temperature), and they could all be easily discriminated (Fig. 4).

It requires 3 to 4 hours to confirm a target gene using a thermal cycler by a standard process from amplification to electrophoresis. However, the real time PCR using the present invention only requires as short as about 40 min for a process from amplification of a target gene to melting curve analysis, and simultaneously enables typing of toxin types.

### Industrial Applicability

The present invention provides a method for totally testing with a common procedure a variety of gene groups which encode TDH-related toxin protein groups having various amino acid sequences. This method enables rapid determination of whether or not bacteria having any gene of TDH-related toxin protein are present in food (in particular, perishable fishes and seafoods to be consumed fresh). When bacteria having toxin genes are present, this method further enables rapid evaluation of food safety, since the number of the existing bacteria having toxin genes can be found with this method. Therefore, the present invention greatly contributes to prevention of food poisoning, and promotion of efficiency and high precision for hygiene control in the fields of food manufacturing and food distribution.

The present invention also enables detection of a gene of TDH-related toxin protein from feces of food poisoning patients and from foods, and rapid typing of the toxin type. Moreover, the present invention enables determination of whether a strain isolated from feces or foods has a gene of TDH-related toxin protein, and if the strain has the gene, enables rapid typing of its toxin type.

Present specification incorporates by reference contents of specification and drawings of patent application number 2001-320403 filed in the Japan Patent Office on October 18, 2001 on which priority is claimed.

## Claims

1. A method for detecting and quantitatively determining pathogenic bacteria having genes of TDH-related toxin [TDH (Thermostable direct hemolysin), TRH (TDH-related hemolysin) and analogous hemolysin protein], which comprises the step of using gene sequences encoding common amino acid sequences being present on the amino acid sequences of thermostable hemolysin protein groups [TDH (Thermostable direct hemolysin), TRH (TDH-related hemolysin) and analogous hemolysin protein, hereinafter referred to as TDH-related toxin] that are widely distributed among pathogenic bacteria of the genus *Vibrio*, thereby widely and totally detecting and quantitatively determining TDH-related toxin genes.

2. A method for totally detecting and discriminating among bacteria having TDH-related toxin genes, which comprises the step of totally amplifying genes of TDH-related toxins widely distributed among pathogenic bacteria of the genus *Vibrio* using a plurality of oligonucleotides as a PCR primer pair which encode part or the whole of at least two amino acid sequences selected from the following amino acid sequences (1) to (7) that are the common amino acid sequences of claim 1, and have functions as a practical primer:
(1) LPS (V or I) PFP (A or S) PGSDE (L or I) LFVVR, (2) KRKPY, (3) Y (M or I) TV (N or S) IN, (4) YTMAA (V or L) SGYK, (5) YLDETP (E or S) YFV, (6) VEAYESG, (7) VMCISNK.

3. A primer for amplifying TDH-related toxin gene, which contains oligonucleotides encoding part of or the whole amino acid sequence selected from the following amino acid sequences (1) to (7) that are the common amino acid sequences of claim 1:
(1) LPS (V or I) PFP (A or S) PGSDE (L or I) LFVVR, (2) KRKPY, (3) Y (M or I) TV (N or S) IN, (4) YTMAA (V or L) SGYK, (5) YLDETP (E or S) YFV, (6 ) VEAYESG, (7) VMCISNK.

4. A primer for amplifying TDH-related toxin gene, which contains 5'-gaygarhtnytnttygtngt-3' encoding DE (L or I) LFVV among amino acid sequence (1) LPS (V or I) PFP (A or S) PGSDE (L or I) LFVVLR, and its corresponding complementary strand.

5. A primer for amplifying TDH-related toxin gene, which contains 5'-ccnswytcrtangcytcnac-3' which is a complementary strand of the nucleotide sequence encoding amino acid sequence (6)VEAYESG, and its corresponding complementary strand.

6. A primer for amplifying TDH-related toxin gene, which contains 5'-tayatgacngtnaayathaayg-3' encoding YMTVNIN among amino acid sequence (3) Y (M or I) TV (N or S) IN and its corresponding complementary strand.

7. A primer for amplifying TDH-related toxin gene, which contains 5'-acraartaytcnggngtytcrtc-3' which is a complementary strand of the nucleotide sequence encoding DETPEYFV among amino acid sequence (5) YLDETP (E or S) YFV and its corresponding complementary strand.

8. A method for totally detecting and quantitatively determining bacteria producing TDH-related toxin, which comprises the step of detecting TDH-related toxin gene using the primers of any one of claims 3 to 7.

9. A method for typing a toxin type encoded by a detected toxin gene, which comprises the steps of totally amplifying a TDH-related toxin gene using gene sequences encoding common amino acid sequences present on the amino acid sequences of TDH and TRH for a gene amplification system; and analyzing Tm value (specific melting temperature) of the amplified products.

10. A method for typing a toxin type produced by TDH-related toxin protein -producing bacteria, wherein the primers of any one of claims 3 to 7 are used.

11. A kit for detecting and quantitatively determining a gene of TDH-related toxin protein and bacteria which produce TDH-related toxin protein, or for testing for typing the toxin type, which contains the primers of any one of claims 3 to 7.


SEQUENCE LISTING

<110> NICHIREI CORPORATION

<120> Method for Detecting and Quantitatively Determining Hemolysin-prod
ucing Bacteria by Totally Detecting and Quantitatively Determining Therm
ostable Direct Hemolysin-related Gene (tdh-related toxin gene) of Food P
oisoning Bacteria

<130> PH-1547PCT

<140> JP 2001-320403
<141> 2001-10-18

<160> 46

<170> PatentIn Ver. 2.1

<210> 1
<211> 149
<212> PRT
<213> Vibrio parahaemolyticus

<400> 1
Phe Glu Leu Pro Ser Val Pro Phe Pro Ala Pro Gly Ser Asp Glu Ile
 1               5                  10                  15

Leu Phe Val Val Arg Asp Thr Thr Phe Asn Thr Asn Ala Pro Val Asn
            20                  25                  30

Val Glu Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys His Gly His Ser Ala Val Phe Val
                85                  90                  95

Lys Ser Asp Gln Val Gln Leu Gln His Ser Tyr Asp Ser Val Ala Asn
            100                 105                 110

Glu Thr Pro Glu Tyr Phe Val Asn Val Glu Ala Tyr Glu Ser Gly Ser
        115                 120                 125

Gly Asn Ile Leu Val Met Cys Ile Ser Asn Lys Glu Ser Phe Phe Glu
    130                 135                 140

Cys Lys His Gln Gln
145

<210> 2
<211> 165
<212> PRT
<213> Vibrio parahaemolyticus

<400> 2
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Asn Ala Pro Val Asn
            20                  25                  30

Val Glu Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys His Gly His Ser Ala Val Phe Val
                85                  90                  95

Lys Ser Asp Gln Val Gln Leu Gln His Ser Tyr Asp Ser Val Ala Ser
            100                 105                 110

Phe Val Gly Glu Asp Gln Asp Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Lys His Gln Gln
                165

<210> 3
<211> 165
<212> PRT
<213> Vibrio parahaemolyticus

<400> 3
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Asn Ala Pro Val Asn
            20                  25                  30

Val Asn Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys Asn Gly His Ser Ala Val Phe Val
                85                  90                  95

Lys Ser Asp Gln Val Gln Leu Gln His Ser Tyr Asn Ser Val Ala Asn

            100                 105                 110

Phe Val Gly Glu Asp Glu Asp Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Lys Tyr Gln Asn
                165

<210> 4
<211> 165
<212> PRT
<213> Vibrio parahaemolyticus

<400> 4
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Asn Ala Pro Val Asn
            20                  25                  30

Val Glu Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys His Gly His Ser Ala Val Phe Val
                85                  90                  95

Lys Ser Asp Gln Val Gln Leu Gln His Ser Tyr Asp Ser Val Ala Ser
            100                 105                 110

Phe Val Gly Glu Asp Glu Asp Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Lys His Gln Gln
                165

<210> 5
<211> 165
<212> PRT
<213> Vibrio parahaemolyticus

<400> 5
Phe Glu Leu Pro Ser Val Pro Phe Pro Ala Pro Gly Ser Asp Glu Ile
1               5                   10                  15
Leu Phe Val Val Arg Asp Thr Thr Phe Asn Thr Gln Ala Pro Val Asn
            20                  25                  30
Val Lys Val Ser Asp Phe Trp Thr Asn Arg Asn Val Lys Arg Lys Pro
        35                  40                  45
Tyr Glu Asp Val Tyr Gly Gln Ser Val Phe Thr Thr Ser Gly Thr Lys
    50                  55                  60
Trp Leu Thr Ser Tyr Met Thr Val Asn Ile Asn Asp Lys Asp Tyr Thr
65                  70                  75                  80
Met Ala Ala Val Ser Gly Tyr Lys Ser Gly His Ser Ala Val Phe Val
                85                  90                  95
Lys Ser Gly Gln Val Gln Leu Gln His Ser Tyr Asn Ser Val Ala Asn
            100                 105                 110
Phe Val Gly Glu Asp Glu Gly Ser Ile Pro Ser Lys Met Tyr Leu Asp
        115                 120                 125
Glu Thr Pro Glu Tyr Phe Val Asn Val Glu Ala Tyr Glu Ser Gly Ser
    130                 135                 140
Gly Asn Ile Leu Val Met Cys Ile Ser Asn Lys Glu Ser Phe Phe Glu
145                 150                 155                 160

Cys Lys His Gln Gln
                165

<210> 6
<211> 165
<212> PRT
<213> Vibrio parahaemolyticus

<400> 6
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Gln Ala Pro Val Asn
            20                  25                  30

Val Lys Val Ser Asp Phe Trp Thr Asn Arg Asn Val Lys Arg Lys Pro
        35                  40                  45

Tyr Glu Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys Ser Gly His Ser Ala Val Phe Val
                85                  90                  95

Lys Ser Asp Gln Val Gln Leu Gln His Ser Tyr Asn Ser Val Ala Asn

            100                 105                 110

Phe Val Gly Glu Asp Glu Gly Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Lys His Gln Gln
                165

<210> 7
<211> 165
<212> PRT
<213> Vibrio parahaemolyticus

<400> 7
Phe Glu Leu Pro Ser Val Pro Phe Pro Ala Pro Gly Ser Asp Glu Ile
1               5                   10                  15

Leu Phe Val Val Arg Asp Ala Thr Phe Asn Thr Asn Ala Pro Val Asn
            20                  25                  30

Val Lys Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys Arg Gly His Ser Ala Val Phe Val
                85                  90                  95

Lys Ser Asp Gln Val Gln Leu Gln His Ser Tyr Asn Ser Val Ala Asn
            100                 105                 110

Phe Val Gly Lys Asp Glu Asp Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Glu His Gln Lys
                165

<210> 8
<211> 165
<212> PRT
<213> Vibrio parahaemolyticus

<400> 8
Phe Glu Leu Pro Ser Val Pro Phe Pro Ala Pro Gly Ser Asp Glu Ile
1               5                   10                  15
Leu Phe Val Val Arg Asp Ala Thr Phe Asn Thr Asn Ala Pro Val Asn
            20                  25                  30
Val Lys Val Ser Asp Phe Trp Thr Asn Arg Asn Val Lys Arg Lys Pro
        35                  40                  45
Tyr Lys Asp Val Tyr Gly Gln Ser Val Phe Thr Thr Ser Gly Thr Lys
    50                  55                  60
Trp Leu Thr Ser Tyr Met Thr Val Asn Ile Asn Asp Lys Asp Tyr Thr
65                  70                  75                  80
Met Ala Ala Val Ser Gly Tyr Lys Arg Gly His Ser Ala Val Phe Val
                85                  90                  95
Lys Ser Asp Gln Val Gln Leu Gln His Ser Tyr Asn Ser Val Ala Asn
            100                 105                 110
Phe Val Gly Glu Asp Glu Asp Ser Ile Pro Ser Lys Met Tyr Leu Asp
        115                 120                 125
Glu Thr Pro Glu Tyr Phe Val Asn Val Glu Ala Tyr Glu Ser Gly Ser
    130                 135                 140
Gly Asn Ile Leu Val Met Cys Ile Ser Asn Lys Glu Ser Phe Phe Glu
145                 150                 155                 160

Cys Glu His Gln Lys
                165

<210> 9
<211> 165
<212> PRT
<213> Vibrio cholerae

<400> 9
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Asn Ala Pro Val Asn
            20                  25                  30

Val Lys Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys Asn Gly His Ser Ala Val Phe Val
                85                  90                  95

Lys Ser Asp Gln Val Gln Leu Gln His Ser Tyr Asn Ser Val Ala Asn

            100                 105                 110

Phe Val Gly Glu Asp Glu Asp Ser Ile Pro Ser Lys Met Tyr Leu Asp
        115                 120                 125

Glu Thr Pro Glu Tyr Phe Val Asp Val Glu Ala Tyr Glu Ser Gly Ser
    130                 135                 140

Gly Asn Ile Leu Val Met Cys Ile Ser Asn Lys Glu Ser Phe Phe Glu
145                 150                 155                 160

Cys Lys Tyr Gln Asn
                165

<210> 10
<211> 165
<212> PRT
<213> Vibrio mimicus

<400> 10
Phe Glu Leu Pro Ser Val Pro Phe Pro Ala Pro Gly Ser Asp Glu Ile
1               5                   10                  15

Leu Phe Val Val Arg Asp Thr Thr Phe Asn Thr Lys Ala Pro Val Asn
            20                  25                  30

Val Lys Val Ser Asp Phe Trp Thr Asn Arg Asp Val Lys Arg Lys Pro
        35                  40                  45

Tyr Glu Asp Val Tyr Gly Gln Ser Val Phe Thr Thr Ser Gly Thr Lys
    50                  55                  60

Trp Leu Thr Ser Tyr Met Thr Val Asn Ile Asn Asp Lys Asp Tyr Thr
65                  70                  75                  80

Met Ala Ala Val Ser Gly Tyr Lys Ser Gly His Ser Ala Val Phe Val
                85                  90                  95

Lys Ser Asp Gln Val Gln Leu Gln His Ser Tyr Asn Ser Val Ala Asn
            100                 105                 110

Phe Val Gly Glu Asp Glu Asp Ser Ile Pro Ser Lys Met Tyr Leu Asp
        115                 120                 125

Glu Thr Pro Glu Tyr Phe Val Asn Val Glu Ala Tyr Glu Ser Gly Ser
    130                 135                 140

Gly Asn Val Leu Val Met Cys Ile Ser Asn Lys Glu Ser Phe Phe Glu
145                 150                 155                 160

Cys Glu His Gln Arg
                165

<210> 11
<211> 165
<212> PRT
<213> Vibrio hollisae

<400> 11
Phe Glu Leu Pro Ser Ile Pro Phe Pro Ser Pro Gly Ser Asp Glu Ile
1               5                   10                  15

Leu Phe Val Val Arg Asp Thr Thr Phe Asn Thr Lys Glu Pro Val Asn
            20                  25                  30

Val Lys Val Ser Asp Phe Trp Thr Asn Arg Asn Val Lys Arg Lys Pro
        35                  40                  45

Tyr Lys Asp Val Tyr Gly Gln Ser Val Phe Thr Thr Ser Gly Ser Lys
    50                  55                  60

Trp Leu Thr Ser Tyr Met Thr Val Ser Ile Asn Asn Lys Asp Tyr Thr
65                  70                  75                  80

Met Ala Ala Val Ser Gly Tyr Lys Asp Gly Phe Ser Ser Val Phe Val
                85                  90                  95

Lys Ser Gly Gln Ile Gln Leu Gln His Tyr Tyr Asn Ser Val Ala Asp
            100                 105                 110

Phe Val Gly Gly Asp Glu Asn Ser Ile Pro Ser Lys Thr Tyr Leu Asp
        115                 120                 125

Glu Thr Pro Ser Tyr Phe Val Asn Val Glu Ala Tyr Glu Ser Gly Ser
    130                 135                 140

Gly Asn Ile Leu Val Met Cys Ile Ser Asn Lys Glu Ser Tyr Phe Glu
145                 150                 155                 160

Cys Glu Glu Gln Gln
                165

<210> 12
<211> 165
<212> PRT
<213> Vibrio hollisae

<400> 12
Phe Glu Leu Pro Ser Ile Pro Phe Pro Ser Pro Gly Ser Asp Glu Ile
1               5                   10                  15

Leu Phe Val Val Arg Asp Thr Thr Phe Asn Thr Lys Glu Pro Val Asn
            20                  25                  30

Val Lys Val Ser Asp Phe Trp Thr Asn Arg Asn Val Lys Arg Lys Pro
        35                  40                  45

Tyr Glu Asp Val Tyr Gly Gln Ser Val Phe Thr Thr Ser Gly Ser Lys
    50                  55                  60

Trp Leu Thr Ser Tyr Met Thr Val Ser Ile Asn Asn Lys Asp Tyr Thr
65                  70                  75                  80

Met Ala Ala Val Ser Gly Tyr Lys Asp Gly Phe Ser Ser Val Phe Val
                85                  90                  95

Lys Ser Gly Gln Ile Gln Leu Gln His Tyr Tyr Asn Ser Val Ala Asp

            100                 105                 110

Phe Val Gly Gly Asp Glu Asn Ser Ile Pro Ser Lys Thr Tyr Leu Asp
        115                 120                 125

Glu Thr Pro Ser Tyr Phe Val Asn Val Glu Ala Tyr Glu Ser Gly Ser
    130                 135                 140

Gly Asn Ile Leu Val Met Cys Ile Ser Asn Lys Glu Ser Tyr Phe Glu
145                 150                 155                 160

Cys Glu Glu Gln Gln
                165

<210> 13
<211> 165
<212> PRT
<213> Vibrio hollisae

<400> 13
Phe Glu Leu Pro Ser Ile Pro Phe Pro Ser Pro Gly Ser Asp Glu Ile
1               5                   10                  15

Leu Phe Val Val Arg Asp Thr Thr Phe Asn Thr Lys Glu Pro Val Asn
            20                  25                  30

Val Lys Val Ser Asp Phe Trp Thr Asn Arg Asn Val Lys Arg Lys Pro
        35                  40                  45

Tyr Lys Asp Val His Gly Gln Ser Val Phe Ile Thr Ser Gly Thr Lys
    50                  55                  60

Trp Leu Thr Ser Tyr Met Thr Val Ser Ile Asn Asn Lys Asp Tyr Thr
65                  70                  75                  80

Met Ala Ala Val Ser Gly Tyr Lys Asp Gly Phe Ser Ser Val Phe Val
                85                  90                  95

Lys Ser Gly Gln Ile Gln Leu Gln His Tyr Tyr Asn Ser Val Ala Asp
            100                 105                 110

Phe Val Gly Gly Asp Glu Asn Ser Ile Pro Ser Lys Thr Tyr Leu Asp
        115                 120                 125

Glu Thr Pro Glu Tyr Phe Val Asn Val Glu Ala Tyr Glu Ser Gly Ser
    130                 135                 140

Gly Asn Ile Leu Val Met Cys Ile Ser Asn Lys Glu Ser Tyr Phe Glu
145                 150                 155                 160

Cys Glu Ser Gln Gln
                165

<210> 14
<211> 165
<212> PRT
<213> Vibrio parahaemolyticus

<400> 14
Ile Asp Leu Pro Ser Ile Pro Phe Pro Ser Pro Gly Ser Asp Glu Leu
1               5                   10                  15

Leu Phe Val Val Arg Asn Thr Thr Ile Lys Thr Glu Ser Pro Val Asn
            20                  25                  30

Ala Ile Val Asn Asp Tyr Trp Thr Asn Arg Asn Ile Lys Arg Lys Pro
        35                  40                  45

Tyr Lys Ser Val His Gly Gln Ser Ile Phe Thr Thr Ser Gly Ser Lys
    50                  55                  60

Trp Leu Ser Ala Tyr Ile Thr Val Asn Ile Asn Gly Asn Asn Tyr Thr
65                  70                  75                  80

Met Ala Ala Leu Ser Gly Tyr Lys Asp Gly Leu Ser Thr Val Phe Thr
                85                  90                  95

Lys Ser Glu Lys Thr Ser Leu Asn Gln Asn Tyr Ser Ser Val Ser Asp
            100                 105                 110

Phe Val Gly Glu Asn Glu Glu Ser Leu Pro Ser Val Thr Tyr Leu Asp
        115                 120                 125

Glu Thr Pro Glu Tyr Phe Val Asn Val Glu Ala Tyr Glu Ser Gly Asn
    130                 135                 140

Gly His Met Phe Val Met Cys Ile Ser Asn Lys Ser Ser Phe Asp Glu
145                 150                 155                 160

Cys Met Ser Gln Asn
                165

<210> 15
<211> 165
<212> PRT
<213> Vibrio parahaemolyticus

<400> 15
Ile Asp Leu Pro Ser Ile Pro Phe Pro Ser Pro Gly Ser Asp Glu Leu
1               5                   10                  15

Leu Phe Val Val Arg Asn Thr Thr Ile Lys Thr Glu Ser Pro Val Asn
            20                  25                  30

Ala Ile Val Asp Asp Tyr Trp Thr Asn Arg Asn Ile Lys Arg Lys Pro
        35                  40                  45

Tyr Lys Ser Val His Gly Gln Ser Ile Phe Thr Thr Ser Gly Ser Lys
    50                  55                  60

Trp Leu Ser Ala Tyr Met Thr Val Asn Ile Asn Gly Asn Asn Tyr Thr
65                  70                  75                  80

Met Ala Ala Leu Ser Gly Tyr Lys Asp Gly Leu Ser Thr Val Phe Thr
                85                  90                  95

Lys Ser Glu Lys Thr Ser Leu Asn Gln Asn Tyr Ser Ser Val Ser Asp

            100                 105                 110

Phe Val Gly Glu Asn Glu Glu Ser Leu Pro Ser Val Thr Tyr Leu Asp
        115                 120                 125

Glu Thr Pro Glu Tyr Phe Val Asn Val Glu Ala Tyr Glu Ser Gly Asn
    130                 135                 140

Gly His Met Phe Val Met Cys Ile Ser Asn Lys Ser Ser Phe Asp Glu
145                 150                 155                 160

Cys Met Ser Gln Asn
                165

<210> 16
<211> 165
<212> PRT
<213> Vibrio parahaemolyticus

<400> 16
Ile Asp Leu Pro Ser Ile Pro Phe Pro Ser Pro Gly Ser Asp Glu Leu
1               5                   10                  15

Leu Phe Val Val Arg Asn Thr Thr Ile Lys Thr Glu Ser Pro Val Lys
            20                  25                  30

Ala Ile Val Glu Asp Tyr Trp Thr Asn Arg Thr Ile Lys Arg Lys Pro
        35                  40                  45

Tyr Lys Asp Val Tyr Gly Gln Ser Val Phe Thr Thr Ala Gly Ser Lys
50                  55                  60

Trp Leu Ser Ala Tyr Met Thr Val Asn Ile Asn Gly His Asn Tyr Thr
65                  70                  75                  80

Met Ala Ala Leu Ser Gly Tyr Lys His Gly Thr Ser Thr Val Phe Thr
                85                  90                  95

Lys Ser Glu Lys Thr Ser Leu Asn Gln Asp Phe Tyr Ser Val Lys Ser
            100                 105                 110

Phe Val Asp Asp Ser Glu Glu Ser Ile Pro Ser Ile Asn Tyr Leu Asp
        115                 120                 125

Glu Thr Pro Glu Tyr Phe Val Thr Val Glu Ala Tyr Glu Ser Gly Asn
    130                 135                 140

Gly His Met Phe Val Met Cys Ile Ser Asn Lys Leu Ser Phe Gly Glu
145                 150                 155                 160

Cys Lys Ser Gln Ile
                165

<210> 17
<211> 19
<212> PRT
<213> Artificial Sequence

<220>
<223> Description of Artificial Sequence:fragment of TDH
      or TRH

<400> 17
Leu Pro Ser Val Pro Phe Pro Ala Pro Gly Ser Asp Glu Leu Leu Phe
 1               5                   10                  15

Val Val Arg

<210> 18
<211> 19
<212> PRT
<213> Artificial Sequence

<220>
<223> Description of Artificial Sequence:fragment of TDH
      or TRH

<400> 18
Leu Pro Ser Val Pro Phe Pro Ala Pro Gly Ser Asp Glu Ile Leu Phe
 1               5                   10                  15

Val Val Arg

<210> 19

<211> 19
<212> PRT
<213> Artificial Sequence

<220>
<223> Description of Artificial Sequence:fragment of TDH
      or TRH

<400> 19
Leu Pro Ser Val Pro Phe Pro Ser Pro Gly Ser Asp Glu Leu Leu Phe
  1               5                  10                  15

Val Val Arg

<210> 20
<211> 19
<212> PRT
<213> Artificial Sequence

<220>
<223> Description of Artificial Sequence:fragment of TDH
      or TRH

<400> 20
Leu Pro Ser Val Pro Phe Pro Ser Pro Gly Ser Asp Glu Ile Leu Phe
  1               5                  10                  15

Val Val Arg

<210> 21
<211> 19
<212> PRT
<213> Artificial Sequence

<220>
<223> Description of Artificial Sequence:fragment of TDH
      or TRH

<400> 21
Leu Pro Ser Ile Pro Phe Pro Ala Pro Gly Ser Asp Glu Ile Leu Phe
  1               5                  10                  15

Val Val Arg

<210> 22
<211> 19
<212> PRT
<213> Artificial Sequence

<220>
<223> Description of Artificial Sequence:fragment of TDH
      or TRH

<400> 22

Leu Pro Ser Ile Pro Phe Pro Ser Pro Gly Ser Asp Glu Leu Leu Phe
1               5                   10                  15

Val Val Arg

<210> 23
<211> 20
<212> PRT
<213> Artificial Sequence

<220>
<223> Description of Artificial Sequence:fragment of TDH
      or TRH

<400> 23
Leu Pro Ser Ile Pro Phe Pro Ala Pro Gly Ser Asp Glu Leu Ile Leu
1               5                   10                  15

Phe Val Val Arg
            20

<210> 24
<211> 19
<212> PRT
<213> Artificial Sequence

<220>

<223> Description of Artificial Sequence:fragment of TDH
      or TRH

<400> 24
Leu Pro Ser Ile Pro Phe Pro Ser Pro Gly Ser Asp Glu Ile Leu Phe
  1               5                  10                  15

Val Val Arg

<210> 25
<211> 5
<212> PRT
<213> Artificial Sequence

<220>
<223> Description of Artificial Sequence:fragment of TDH
      or TRH

<400> 25
Lys Arg Lys Pro Tyr
  1               5

<210> 26
<211> 7
<212> PRT
<213> Artificial Sequence

<220>
<223> Description of Artificial Sequence:fragment of TDH
      or TRH

<400> 26
Tyr Met Thr Val Asn Ile Asn
  1               5

<210> 27
<211> 7
<212> PRT
<213> Artificial Sequence

<220>
<223> Description of Artificial Sequence:fragment of TDH
      or TRH

<400> 27
Tyr Met Thr Val Ser Ile Asn
  1               5

<210> 28
<211> 7
<212> PRT
<213> Artificial Sequence

<220>
<223> Description of Artificial Sequence:fragment of TDH

or TRH

<400> 28
Tyr Ile Thr Val Asn Ile Asn
1               5

<210> 29
<211> 7
<212> PRT
<213> Artificial Sequence

<220>
<223> Description of Artificial Sequence:fragment of TDH
      or TRH

<400> 29
Tyr Ile Thr Val Ser Ile Asn
1               5

<210> 30
<211> 10
<212> PRT
<213> Artificial Sequence

<220>
<223> Description of Artificial Sequence:fragment of TDH
      or TRH

<400> 30
Tyr Thr Met Ala Ala Val Ser Gly Tyr Lys
1               5                   10

<210> 31
<211> 10
<212> PRT
<213> Artificial Sequence

<220>
<223> Description of Artificial Sequence:fragment of TDH
      or TRH

<400> 31
Tyr Thr Met Ala Ala Leu Ser Gly Tyr Lys
1               5                   10

<210> 32
<211> 10
<212> PRT
<213> Artificial Sequence

<220>
<223> Description of Artificial Sequence:fragment of TDH
      or TRH

<400> 32
Tyr Leu Asp Glu Thr Pro Glu Tyr Phe Val

1                5                    10

<210> 33
<211> 10
<212> PRT
<213> Artificial Sequence

<220>
<223> Description of Artificial Sequence:fragment of TDH
      or TRH

<400> 33
Tyr Leu Asp Glu Thr Pro Ser Tyr Phe Val
 1               5                   10

<210> 34
<211> 7
<212> PRT
<213> Artificial Sequence

<220>
<223> Description of Artificial Sequence:fragment of TDH
      or TRH

<400> 34
Val Glu Ala Tyr Glu Ser Gly
 1               5

<210> 35
<211> 7
<212> PRT
<213> Artificial Sequence

<220>
<223> Description of Artificial Sequence:fragment of TDH
or TRH

<400> 35
Val Met Cys Ile Ser Asn Lys
1               5

<210> 36
<211> 20
<212> DNA
<213> Artificial Sequence

<220>
<223> Description of Artificial Sequence:Primer for gene
coding TDH, TRH, or similar haemolysin

<220>
<221>
<222> 9, 12, 18
<223> n represents a, g, c or t

<400> 36

gaygarhtny tnttygtngt                                                    20

<210> 37
<211> 20
<212> DNA
<213> Artificial Sequence

<220>
<223> Description of Artificial Sequence:Primer for gene
      coding TDH, TRH, or similar haemolysin

<220>
<221>
<222> 3, 12, 18
<223> n represents a, g, c or t

<400> 37
ccnswytcrt angcytcnac                                                    20

<210> 38
<211> 23
<212> DNA
<213> Artificial Sequence

<220>
<223> Description of Artificial Sequence:Primer for gene
      coding TDH, TRH, or similar haemolysin

<220>
<221>
<222> 12, 15
<223> n represents a, g, c or t

<400> 38
acraartayt cnggngtytc rtc                                                23

<210> 39
<211> 22
<212> DNA
<213> Artificial Sequence

<220>
<223> Description of Artificial Sequence:Primer for gene
      coding TDH, TRH, or similar haemolysin

<220>
<221>
<222> 9, 12
<223> n represents a, g, c or t

<400> 39
tayatgacng tnaayathaa yg                                                 22

<210> 40
<211> 353
<212> DNA

<213> Vibrio parahaemolyticus IFO12711 T

<400> 40
gttagaaata caacaatcaa aactgaatcc ccggttaagg caattgtgga ggactattgg 60
acaaaccgaa acataaaaag aaaaccatac aaagatgtat acggtcaatc ggttttcaca 120
acagcaggtt caaagtggtt aagcgcctat atgacagtaa acatcaatgg tcataactat 180
acgatggcag ctctttctgg ttataaagat ggtatttcta cggtcttcac aaaatcagag 240
aaaacaagcc taaagcaaga ctattcctcg gtaaaatatt ttgttgatga caacgaagaa 300
tcaataccaa gtgtaactta tttagatgaa acatcagaat actttgttac tgt 353

<210> 41
<211> 353
<212> DNA
<213> Vibrio parahaemolyticus V89 655

<400> 41
gttagaaata caacaataaa aactgaatca ccagttaacg caatcgttaa tgactactgg 60
acaaaccgaa acataaaacg aaaaccatat aaaagcgttc acggtcaatc tattttcacg 120
acttcaggct caaaatggtt aagcgcctat ataacggtaa atattaatgg aaataactac 180
acaatggctg ctctttctgg ctataaagat ggcctttcaa cggtcttcac aaaatcagaa 240
aaaacaagcc taaatcagaa ctattcttct gttagtgatt tcgttggtga aaatgaagaa 300
tcattgccaa gtgtaacgta tttggatgaa acgccagaat atttcgtcaa tgt 353

<210> 42
<211> 353
<212> DNA
<213> Vibrio parahaemolyticus V89 656

<400> 42

gttcgagata caacttttaa tacccaagct ccggtcaatg taaaggtctc tgacttttgg 60

acaaaccgta atgtaaaaag aaaaccgtac aaagatgttt atggtcaatc agtattcaca 120

acgtcaggta ctaaatggct gacatcctac atgactgtga acattaatga taaagactat 180

acaatggcag cggtgtctgg ctataagcgc ggtcattctg ctgtgttcgt aaaatcagat 240

caagtacagc ttcaacattc ctatgattct gtagctaact ttgttggtga agatgaaggt 300

tctattccaa gtaaaatgta tttggatgaa actccagaat attttgttaa tgt 353

<210> 43

<211> 353

<212> DNA

<213> Vibrio parahaemolyticus V99 157

<400> 43

gttcgagata caacttttaa tacccaagct ccggtcaatg taaaggtctc tgacttttgg 60

acaaaccgta atgtaaaaag aaaaccgtac aaagatgttt atggtcaatc agtattcaca 120

acgtcaggta ctaaatggtt gacatcctac atgactgtga acattaatga taaagactat 180

acaatggcag cggtgtctgg ctataagaac ggtcattctg ctgtgttcgt aaaatcagat 240

caagtacagc ttcaacattc ctatgattct gtagctaact ttgttggtga agatgaaggt 300

tctattccaa gtaaaatgta tttggatgaa actccagaat attttgttaa tgt 353

<210> 44

<211> 353

<212> DNA

<213> Vibrio parahaemolyticus V99 161

<400> 44
gttcgagata caacttttaa tacccaagct ccggtcaatg taaaggtctc tgacttttgg 60
acaaaccgta atgtaaaaag aaaaccgtac aaagatgttt atggtcaatc agtattcaca 120
acgtcaggta ctaaatggct gacatcctac atgactgtga acattaatga taaagactat 180
acaatggcag cggtgtctgg ctataagcgc ggtcattctg ctgtgttcgt aaaatcagat 240
caagtacagc ttcaacattc ctatgattct gtagctaact ttgttggtga agatgaaggt 300
tctattccaa gtaaaatgta tttggatgaa actccagaat attttgttaa tgt 353

<210> 45
<211> 353
<212> DNA
<213> Vibrio parahaemolyticus V99 177

<400> 45
gttcgagata caacttttaa tacccaagct ccggtcaatg taaaggtctc tgacttttgg 60
acaaaccgta atgtaaaaag aaaaccgtac aaagatgttt atggtcaatc agtattcaca 120
acgtcaggta ctaaatggct gacatcctac atgactgtga acattaatga taaagactat 180
acaatggcag cggtgtctgg ctataagagc ggtcattctg ctgtgttcgt aaaatcagat 240
caagtacagc ttcaacattc ctatgattct gtagctaact ttgttggtga agatgaaggt 300
tctattccaa gtaaaatgta tttggatgaa actccagaat attttgttaa tgt 353

<210> 46
<211> 353
<212> DNA
<213> Vibrio parahaemolyticus V99 223

<400> 46

gttcgagata caacttttaa tacccaagct ccggtcaatg taaaggtctc tgacttttgg 60
acaaaccgta atgtaaaaag aaaaccgtac aaagatgttt atggtcaatc agtattcaca 120
acgtcaggta ctaaatggct gacatcctac atgactgtga acattaatga taaagactat 180
acaatggcag cggtgtctgg ctataagcgc ggtcattctg ctgtgttcgt aaaatcagat 240
caagtacagc ttcaacattc ctatgattct gtagctaact ttgttggtga agatgaaggt 300
tctattccaa gtaaaatgta tttggatgaa actccagaat attttgttaa tgt 353